Europäisches Patentamt

European Patent Office

Office européen des brevets

(19)

(11) Veröffentlichungsnummer: **0 220 548**
A2

# (12) EUROPÄISCHE PATENTANMELDUNG

(21) Anmeldenummer: 86113788.3

(22) Anmeldetag: 04.10.86

(51) Int. Cl.⁴: **C 12 N 1/04,** C 12 N 1/38,
A 23 C 9/13, A 23 B 4/12,
C 12 C 11/00

(30) Priorität: 21.10.85 LU 86129

(43) Veröffentlichungstag der Anmeldung: 06.05.87
Patentblatt 87/19

(84) Benannte Vertragsstaaten: **AT BE CH DE ES FR GB GR
IT LI LU NL SE**

(71) Anmelder: **CHIMICASA GMBH, Wiesentalstrasse 81,
CH-7000 Chur (CH)**

(72) Erfinder: **Lembke, Andreas, Prof. Dr. Dr. med.,
Eutinerstrasse 1, D-2420 Eutin-Sielbeck (DE)**
Erfinder: **Deininger, Rolf, Dr., Fürst-Pückler-Strasse 44,
D-5000 Köln 41 (DE)**
Erfinder: **Lembke, Jürgen, Dr., Eutinerstrasse 1,
D-2420 Eutin-Sielbeck (DE)**

(74) Vertreter: **Hach, Hans Karl, Dr., Tarunstrasse 23,
D-6950 Mosbach-Waldstadt (DE)**

(54) Verfahren zum Aufbereiten von Lebensmitteln und Präparat zum Schützen von zur Lebensmittelaufbereitung dienenden Mikroorganismen.

(57) Bei der Aufbereitung von Lebensmitteln unter Einsatz von Mikroorganismen werden diese durch Zugabe von Ameisensäure oder Ameisensäureester oder Salzen der Ameisensäure und/oder Tetrahydrofolsäure vor Phagenbefall direkt geschützt.

Ein indirekter Schutz wird beschrieben durch Inaktivierung der Bakterienviren in der Umwelt.

EP 0 220 548 A2

## VERFAHREN ZUM AUFBEREITEN VON LEBENSMITTELN UND PRÄPARAT ZUM SCHÜTZEN VON ZUR LEBENSMITTELAUFBEREITUNG DIENENDEN MIKROORGANISMEN

Die Erfindung betrifft ein Verfahren zum Aufbereiten von Lebensmitteln, mittels Starterkultur von Mikroorganismen hergestellt, die zum Zeitpunkt des Bedarfs aktiviert, zu einer Betriebskultur vermehrt oder direkt dem umzusetzenden Substrat zugegeben wird, dergestalt daß die Mikroorganismen gegen Phagenbefall durch Einsatz eines viruziden Präparats in einer für die Fermentation eingesetzten Bakterien und für den Genuß der Lebensmittel unschädlichen Dosis geschützt werden und ein Präparat zur Ausübung dieses Verfahrens.

Die industrielle Lebensmittelaufbereitung, wie zum Beispiel Vergärung, Verkäsung, Fermentierung, erfolgt unter Mitwirkung von Mikroorganismen, nämlich Bakterien, Hefen, Pilzen oder Algen. Diese Mikroorganismen unterliegen dem Befall von Phagen, wodurch die mit den Mikroorganismen angestrebten Umsetzungsprozesse gestört werden können. Aus diesem Grunde ist es wünschenswert, dem Phagenbefall entgegenzuwirken.

Aus der EU-B1-3318 ist ein Verfahren der eingangs genannten Art bekannt, bei dem als viruzides Präparat Schwarzer Pfeffer-Öl eingesetzt wird. In manchen Fällen ist Schwarzer Pfeffer-Öl als Zugabe unerwünscht. Es ist deshalb Aufgabe der Erfindung, ein Verfahren der eingangs genannten Art so auszugestalten, daß es mit einem Zusatz durchführbar ist, der möglichst unschädlich ist und in dem behandelten Lebensmittel auch sonst keine Störungen verursacht.

.Die Erfindung ist dadurch gekennzeichnet, daß das
**viruzide** Präparat mindestens einen zehnprozentigen
**Anteil** an Ameisensäure und/oder Ameisensäureester,
vorzugsweise Ameisensäureethylester, und/oder Salze
der Ameisensäure, und/oder Tetrahydrofolsäure enthält
und/oder daß der Rest des viruziden Präparats aus
einer oder mehreren der nachfolgenden Substanzen
besteht: Schwarzer Pfeffer-Öl, Zimtblüten-Öl,
Cardamom-Öl, Linallylacetat, Zimtaldehyd, Zimtsäureethylester, Safrol, Carvon und cis/trans Citral.

Es ist bekannt, daß Ameisensäure toxische Wirkung auf
Mikroorganismen ausübt. Die viruzide Wirkung der Ameisensäure, Ameisensäureester, Salze der Ameisensäure, Ameisensäureethylester und Tetrahydrofolsäure, die sich die Erfindung zunutze macht, ist jedoch nicht bekannt. Ameisensäure zeigt ebenso wie die anderen möglichen zusätzlichen
Inhaltsstoffe zum viruziden Präparat aufgeführten Substanzen eine viruzide Wirkung, also auch eine phagenschädigende Wirkung, bereits bei einer Konzentration,
die um mehrere Zehnerpotenzen niedriger liegt als diejenige Konzentration bei der Ameisensäure, Ameisensäureester, Ameisensäureethylester, Salze der Ameisensäure
und Tetrahydrofolsäure, die bekannte toxische Wirkung
auf Mikr oorganismen zeigt. Diese breite Spanne bietet
einen bei der Dosierung vorteilhaften Toleranzspielraum,
innerhalb dessen die angestrebte viruzide Wirkung erzielbar ist, ohne dabei Gefahr zu laufen, daß gleichzeitig
die Mikroorganismen, die vor den Phagen geschützt werden
sollten, durch die Ameisensäure, Ameisensäureester,
Ameisensäureethylester, Salze der Ameisensäure und
Tetrahydrofolsäure geschädigt werden.

Ameisensäure und Tetrahydrofolsäure ist für den menschlichen Organismus in den verwendeten Dosen praktisch ungiftig und deshalb unbedenklich als Zusatz für Lebensmittel geeignet. Da die angestrebte viruzide Wirkung bei verhältnismäßig geringen Zusatzmengen von Ameisensäure, Ameisensäureethylester und Tetrahydrofolsäure erzielbar ist, ist auch eine geschmackliche Beeinträchtigung der Lebensmittel durch die zugesetzte Ameisensäure und Ameisensäureethylester nicht in Kauf zu nehmen. Das gilt bedingt auch für die anderen angegebenen möglichen Inhaltsstoffe des viruziden Präparates.

Vorzugsweise wird Ameisensäure und/oder Ameisensäureester, vorzugsweise Ameisensäureethylester, und/oder Salze der Ameisensäure, und/oder Tetrahydrofolsäure als alleiniger Wirkstoff, also zu 100 % (Prozent), im viruziden Präparat eingesetzt.

Ausreichend für den angestrebten Zweck und unschädlich für die beteiligten Mikroorganismen ist es, wenn 25 bis 500 mg viruzider Wirkstoff oder Kombination auf 1 kg Starterkultur eingesetzt wird. Der oder die Wirkstoffe werden in Wasser oder Alkohol zu 1 - 10 % vorverdünnt.

Man kann zum Schutz der Mikroorganismen das viruzide Präparat der Starterkultur und/oder der Betriebskultur und/oder dem Lebensmittel zusammen mit der Betriebskultur zusetzen. Im allgemeinen kann man aber davon ausgehen, daß die Starterkulturen, dann, wenn sie industriell hergestellt werden, steril sind und auch steril aufbewahrt werden. Das kann man unterstützen durch Zugabe des viruziden Präparates in die Starterkultur. Wenn die Starterkultur in der Nahrungsmittelindustrie hergestellt wird, empfiehlt es sich im allgemeinen, auch
die Starterkultur schon mit dem viruziden Präparat
zu schützen. Besonders problematisch wegen der nur
schwer einzuhaltenden Sterilbedingungen ist die
Herstellung der Betriebskultur, die im allgemeinen
in der Industrie vorgenommen wird. Deshalb wird das
viruzide Präparat vorzugsweise beim Ansetzen oder in
Verbindung mit der Betriebskultur eingesetzt.

Wenn die Betriebskultur in einem geschlossenen Tank
angesetzt wird, läßt es sich in vielen Fällen nicht
vermeiden, daß mit Phagen infizierte Außenluft zudringt.
Diesem Umstand trägt eine Weiterbildung der Erfindung
Rechnung, die dadurch gekennzeichnet ist, daß zur Herstellung einer Betriebskultur ein mit Druckausgleich
versehener und im übrigen geschlossener, verschließbare Öffnungen aufweisender Tank durch gespannten Dampf
sterilisiert wird, daß in den erhitzten sterilisierten
Tank erhitzte Milch eingeführt wird, in der möglicherweise noch vorhandene Phagen thermisch inaktiviert
sind, daß während der Abkühlphase das viruzide Präparat mittels eines Vernebelungsgerätes unter Nutzung
der Druckdifferenz zwischen Außen- und Innenraum des
Tanks eingesprüht wird,
daß nach erfolgter Abkühlung die aktive Starterkultur
mit einer geeigneten Vorrichtung in den Tank eingeführt,
durchmischt und dort zur Betriebskultur vemehrt wird,
und daß die mit dem viruziden Präparat behandelte
Starterkultur mit einer geeignten Vorrichtung aseptisch
in den Tank eingeführt wird.

Durch das viruzide Präparat wird die Gasfüllung des
abkühlenden Behälters mit dem verdampften viruziden
Präparat vermischt und damit viruzid. Gas beziehungsweise Luft, das/die zum Druckausgleich beim Abkühlen
zusätzlich in den Tank eindringt und im allgemeinen
nicht steril ist, wird dadurch hinsichtlich seines/ihres
Phagengehaltes sterilisiert.

Die Erfindung betrifft auch ein Präparat zum Schützen
von zur Lebensmittelaufbereitung dienender Mikroorganismen gegen Befall von Phagen. Ein solches
Präparat ist gekennzeichnet durch Ameisensäure und/oder
Ameisensäureester, vorzugsweise Ameisensäureethylester,
und/oder Salze der Ameisensäure, und/oder Tetrahydrofolsäure, eingemischt in eine Trägersubstanz im
Mischungsverhältnis 1:10 bis 1:1000, wobei die Trägersubstanz in das Lebensmittel gut einmischbar und als
unschädlicher Lebensmittelzusatz geeignet ist.

Statt reiner Ameisensäure kann man bei einem solchen
Präparat auch die oben erwähnten, aus Gewürzpflanzen
gewinnbaren, Substanzen einsetzen. Ein entsprechendes
Präparat ist dadurch gekennzeichnet, daß eine Ameisensäuremischung im Mischungsverhältnis 1:10 bis 1:1000
in eine Trägersubstanz eingemischt ist, wobei die
Trägersubstanz in das Lebensmittel gut einmischbar
ist und als unschädlicher Lebensmittelzusatz geeignet
ist und daß diese Ameisensäuremischung aus Ameisensäure
und/oder Ameisensäureester, vorzugsweise Ameisensäureethylester, und/oder Salze der Ameisensäure, und/oder
Tetrahydrofolsäure besteht, der in gewichtsbezogenem Mischungsverhältnis von 9:1 bis 1:9 eine oder
mehrere der nachfolgenden, aus Gewürzpflanzen
gewinnbaren Substanzen, Schwarzer Pfeffer-öl,
Zimtblüten-öl, Cardamom-öl, Linallylacetat,
Zimtaldehyd, Zimtsäureethylester, Safrol, Carvon
und cis/trans Citral untermischt sind.

Versuche haben ergeben, daß die unschädliche Dosis
des viruziden Präparates bei den meisten nützlichen
Mikroorganismen 1:1000, bei einigen sogar 1:100,
betragen kann. Als viruzide Dosis reicht dagegen,
bezogen auf die schützende Masse, bereits eine
Beimischrate von 1:2000 bis 1:100.000. Das ergibt
einen Spielraum von mehreren Zehnerpotenzen, innerhalb der bequem dosiert werden kann mit sicherer
viruzider Wirkung ohne Mikroorganismen zu gefährden.

Man kann aufgrund des großen Spielraums der Starterkultur eine hohe unschädliche Dosis des viruziden
Präparates beimischen. Diese Beimischung wird dann
in der Betriebskultur verdünnt, und aufgrund des
großen Spielraums kann man die Verhältnisse leicht
so festlegen, daß die durch die Verdünnung sich
ergebende reduzierte Beimischrate des viruziden
Präparates in der Betriebskultur noch für die angestrebte viruzide Wirkung ausreicht, sodaß man in die
Betriebskultur kein zusätzliches viruzides Präparat
eingeben muß.

- 7 -

Die Erfindung ist unter anderem anwendbar bei der
Aufbereitung von Milch zu Sauermilch, Joghurt oder
Käse unter Verwendung von Streptococcus lactis,
Streptococcus cremoris, Streptococcus diacetylactis,
Leuconostoc cremoris, Streptococcus thermophilus
oder Lactobacillus bulgaricus;

bei der Vergärung von Bierwürze unter Verwendung einer
Saccaromyces cerevisiae und

zur Aufbereitung von Sauerkraut, Gurken, Kürbissen,
Möhren, Bohnen, anderen Essigprodukten und dergleichen
unter Einsatz dafür geeigneter Mikroorganismen.

Die Erfindung wird nun anhand einiger Beispiele näher
erläutert.

- 12 -
8

- 8 -

## BEISPIEL 1

*Zur Herstellung von Joghurt wird eine Starterkultur von Streptococcus thermophilus und Lactobacillus bulgaricus angesetzt. In die Starterkultur wird als viruzides Präparat maximal 500 mg Ameisensäure auf 1 kg Starterkultur in Form einer zehnprozentigen Lösung eingemischt. Dann wird die Starterkultur portioniert tiefgefroren aufbewahrt.*

*Bei Bedarf wird eine Portion der Starterkultur aufgetaut und unter Sterilbedingungen vorsterilisierter warmer Milch untermischt. Aus der Mischung wird die Betriebskultur gebildet. Die Betriebskultur wird dann, aufgeteilt in Portionen in den erforderlichen Konzentrationen angewärmter Milch zugesetzt, und es wird nach Bebrütung Joghurt gebildet.*

*Das viruzide Präparat ist in der Starterkultur in einer Dosierung enthalten, durch die Mikroorganismen noch nicht geschädigt werden. In der Betriebskultur ist das viruzide Präparat nur soweit verdünnt, daß noch eine ausreichende viruzide Wirkung erhalten bleibt.*

BEISPIEL 2

*Wie Beispiel 1 mit dem einzigen Unterschied, daß das viruzide Präparat der Starterkultur vor der Zugabe in die Betriebskultur zugesetzt wird.*

BEISPIEL 3

*Zur Herstellung von Joghurt wird eine Starterkultur von Streptococcus thermophilus und Lactobacillus bulgaricus angesetzt. Dann wird die Starterkultur in Portionen tiefgefroren aufbewahrt.*

*In einem 1.300 l (Liter) fassenden, mit verschließbaren Öffnungen versehen, im übrigen geschlossenen Tank, wird mit gespanntem Dampf sterilisiert. Es werden 1.000 l (Liter) erhitzte Milch, in der möglicherweise vorhandene Phagen thermisch inaktiviert sind, in den Tank eingefüllt. Während der Abfüllphase wird der überstehende Luftraum (z.B. 200 l) mit dem als Aerosol vorliegenden viruziden Präparat gesättigt in der Weise, daß das viruzide Präparat, z.B. 2 l (Liter) einer 0,2 %igen Lösung, mittels eines Vernebelungsgerätes unter Nutzung der Druckdifferenz zwischen Außen- und Innenraum des Tanks eingespritzt wird. Die Starterkultur oder die mit dem viruziden Präparat behandelte Starterkultur wird mittels einer geeigneten Vorrichtung aseptisch in den Tank eingeführt. Die Tankfüllung wird gemischt und ca. 4 Stunden bei 40 °C stehengelassen. Dadurch wird die Milch umgesetzt in das Endprodukt Joghurt.*

## BEISPIEL 4

Ameisensäure wird im Gewichtsverhältnis 1:50 in
Wasser gelöst. Die Lösung wird im Autoklaven sterilisiert. Das so gewonnene Präparat wird dem durch
Mikroorganismen aufzubereitenden Lebensmittel zugesetzt im Gewichtsverhältnis 2,5 g bis 25 g Präparat
auf 1 kg Lebensmittel, und das entspricht 50 bis 500 mg
Ameisensäure auf 1 kg Lebensmittel.

Die vorgenannten Beispiele sind anwendbar auf andere
Mikroorganismen, nämlich beispielsweise Streptococcus
cremoris, Streptococcus diacetylactis, Leuconostoc
cremoris, Streptococcus thermophilus oder Lactobacillus
bulgaricus. Sie sind anwendbar auf andere Lebensmittel,
nämlich beispielsweise bei der Vergärung von Bierwürze
unter Verwendung einer Saccaromyces cerevisiae und zur
Aufbereitung von Sauerkraut, Gurken, Kürbissen, Möhren,
Bohnen, anderen Essigprodukten und dergleichen unter
Einsatz dafür geeigneter Mikroorganismen.

Sie sind auch abänderbar hinsichtlich der eingesetzten
viruziden Präparate. Es kann das eingesetzte viruzide
Präparat statt aus Ameisensäure auch aus Ameisensäureester oder insbesondere Ameisensäureethylester, Salzen
der Ameisensäure und/oder Tetrahydrofolsäure bestehen
oder aus Mischungen von Ameisensäure und Ameisensäureester. Außerdem kann das viruzide Präparat neben
Ameisensäure beziehungsweise Ameisensäureester eine
oder mehrere der eingangs genannten Wirkstoffe enthalten, mit einer Beimischrate, wie eingangs dargelegt.

**PATENTANSPRÜCHE:**

1. *Verfahren zum Aufbereiten von Lebensmitteln,*
   *bei dem eine Starterkultur der Mikroorganismen*
   *hergestellt und in aufbewahrungsfähigen Zustand*
   *versetzt wird,*
   *bei dem die Starterkultur zum Zeitpunkt des Bedarf*
   *reaktiviert und zu einer Betriebskultur vermehrt*
   *wird,*
   *bei dem die Betriebskultur dem aufzubereitenden*
   *Lebensmittel angeimpft wird und bei der Animpfung*
   *und danach für die Dauer der erwünschten Einwirkung*
   *in dem Lebensmittel der angestrebten Einwirkung ent-*
   *sprechende Lebensbedingungen für die angeimpften*
   *Mikroorganismen aufrechterhalten werden und*
   *bei dem die Mikroorganismen gegen Phagenbefall*
   *durch Einsatz eines viruziden Präparates in einer*
   *für die Mikroorganismen unschädlichen und für die*
   *Lebensmittel und deren Genuß unschädlichen Dosis*
   *geschützt werden, dadurch gekennzeichnet,*
   *daß das viruzide Präparat mindestens einen 10%igen*
   *Anteil an Ameisensäure und/oder Ameisensäureester,*
   *vorzugsweise Ameisensäureethylester, und/oder Salze*
   *der Ameisensäure, und/oder Tetrahydrofolsäure*
   *enthält, und*
   *daß der Rest des viruziden Präparates aus einer oder*
   *mehreren der nachfolgenden Substanzen besteht:*
   *Schwarzer Pfeffer-Öl, Zimtblüten-Öl, Cardamom-Öl,*
   *Linallylacetat, Zimtaldehyd, Zimtsäureethylester,*
   *Safrol, Carvon und cis/trans Citral.*

- 2 -

- 2 -

2. Verfahren nach Anspruch 1, dadurch gekennzeichnet,
daß das viruzide Präparat aus Ameisensäure und/oder
Ameisensäureester, vorzugsweise Ameisensäureethylester, und/oder Salzen der Ameisensäure, und/oder
Tetrahydrofolsäure besteht.

3. Verfahren nach einem der vorhergehenden Ansprüche,
dadurch gekennzeichnet,
daß 25 bis 500 mg (Milligramm) viruzider Wirkstoff
auf 1 kg (Kilogramm) Starterkultur eingesetzt wird.
Der oder die Wirkstoffe werden vorverdünnt zu
1 - 10 % in Alkohol und/oder Wasser.

4. Verfahren nach einem der vorhergehenden Ansprüche,
dadurch gekennzeichnet,
daß das viruzide Präparat der Starterkultur zugesetzt wird.

5. Verfahren nach einem der Ansprüche 1 bis 3, dadurch
gekennzeichnet,
daß das viruzide Präparat erst beim Herstellen der
Betriebskultur eingesetzt wird.

6. Verfahren nach Anspruch 5, dadurch gekennzeichnet,
daß zur Herstellung einer Betriebskultur ein mit
Druckausgleich versehener und im übrigen geschlossener, verschließbare Öffnungen aufweisender Tank
durch gespannten Dampf sterilisiert wird,
daß in den erhitzten sterilisierten Tank erhitzte
Milch eingeführt wird, in der möglicherweise noch
vorhandene Phagen thermisch inaktiviert sind,
.. daß während der Abkühlphase das viruzide Präparat
mittels eines Vernebelungsgerätes unter Nutzung der
Druckdifferenz zwischen Außen- und Innenraum des

- 3 -

- 3 -

Tanks eingesprüht wird,
daß nach erfolgter Abkühlung die aktive Starterkultur
mit einer geeigneten Vorrichtung aseptisch in den Tank
eingeführt wird, durchmischt und dort zur Betriebskultur vermehrt wird und
daß die mit dem viruziden Präparat behandelte Starterkultur mit einer geeigneten Vorrichtung aseptisch in den
Tank eingeführt wird.

7. Verfahren nach einem der vorhergehenden Ansprüche,
gekennzeichnet durch die Anwendung z.B. bei einer
Aufbereitung von Milchprodukten unter Verwendung
geeigneter Bakterien.

8. Präparat zum Schützen von zur Lebensmittelaufbereitung
dienenden Mikroorganismen gegen Befall von Phagen, nach
Anspruch 2, gekennzeichnet durch Ameisensäure und/oder
Ameisensäureester, vorzugsweise Ameisensäureethylester,
und/oder Salze der Ameisensäure, und/oder Tetrahydrofolsäure, eingemischt in eine Trägersubstanz im Mischungsverhältnis 1:10 bis 1:1000, wobei die Trägersubstanz in
das Lebensmittel gut einmischbar und als unschädlicher
Lebensmittelzusatz geeignet ist.

9. Präparat zum Schützen von zur Lebensmittelaufbereitung
dienenden Mikroorganismen gegen Befall von Phagen, nach
Anspruch 1, dadurch gekennzeichnet, daß eine Ameisensäuremischung im Mischungsverhältnis 1:10 bis 1:1000 in
eine Trägersubstanz eingemischt ist, wobei die Trägersubstanz in das Lebensmittel gut einmischbar ist und
als unschädlicher Lebensmittelzusatz geeignet ist, und

- 4 -

P 50 79A 0220548

- 4 -

daß *diese Ameisensäuremischung aus Ameisensäure und/oder Ameisensäureester und/oder Salze der Ameisensäure, vorzugsweise Ameisensäureethylester und/oder Tetrahydrofolsäure besteht, der in gewichtsbezogenem Mischungsverhältnis von 9:1 bis 1:9 eine oder mehrere der nachfolgenden, aus Gewürzpflanzen gewinnbaren Substanzen Schwarzer Pfeffer-Öl, Zimtblüten-Öl, Cardamom-Öl, Linallylacetat, Zimtaldehyd, Zimtsäureethylester, Safrol, Carvon und cis/trans Citral untermischt sind.*

10. *Präparat nach Anspruch 8 oder 9, gekennzeichnet durch Aethanol und/oder Wasser als Trägersubstanz.*

- 5 -